# EUROPEAN PATENT SPECIFICATION

(11) **EP 4 108 658 B1**
(45) Date of publication and mention of the grant of the patent: **04.11.2026**
(21) Application number: 21757166.0
(22) Date of filing: 19.02.2021
(51) Int. Cl.: C07D 311/66, C07C 211/27, C07C 231/12, A61K 31/122, A61P 25/18

(54) **SYNTHESIS OF R-TROLOX SALT, SYNTHESIS OF (R)-2-HYDROXY-2-METHYL-4-(2,4,5-TRIMETHYL-3,6-DIOXOCYCLOHEXA-1,4-DIENYL)BUTANAMIDE FROM R-TROLOX, AND R-TROLOX R-PHENYLETHYLAMINE SALT AND SOLVATES THEREOF**
SYNTHESE EINES R-TROLOX SALZES, SYNTHESE VON (R)-2-HYDROXY-2-METHYL-4-(2,4,5-TRIMETHYL-3,6-DIOXOCYCLOHEXA-1,4-DIENYL)BUTANAMID AUSGEHEND VON R-TROLOX, UND R-TROLOX R-PHENYLETHYLAMIN SALZ UND SOLVATE DAVON
SYNTHÈSE D'UN SÉL DE R-TROLOX, SYNTHÈSE DU (R)-2-HYDROXY-2-MÉTHYL-4-(2,4,5-TRIMÉTHYL-3,6-DIOXOCYCLOHÉXA-1,4-DIÉNYL)BUTANAMIDE Á PARTIR DE R-TROLOX, ET SÉL DE R-TROLOX R-PHÉNYLÉTHYLAMINE ET LEURS SOLVATES

(30) Priority: 21.02.2020 JP 2020028614
(43) Date of publication of application: 28.12.2022
(73) Proprietor: PTC Therapeutics, Inc., Warren, NJ 07059 (US)
(72) Inventor: KURODA, Kiichi, Osaka-shi, Osaka 554-0022 (JP); SAWAMURA, Kiyoto, Osaka-shi, Osaka 554-0022 (JP); TSUYUMINE, Shinjiro, Osaka-shi, Osaka 554-0022 (JP)
(74) Representative: Petty, Catrin Helen
(86) International application number: PCT/JP2021/006465
(87) International publication number: WO 2021/167095

(56) References cited:
- EP-A1- 2 351 729
- WO-A1-02/12221
- WO-A1-2014/168103
- WO-A1-2016/100576
- WO-A1-2016/100579
- JP-A- 2002 167 381
- JP-A- 2011 503 005
- JP-A- 2012 025 705
- JP-A- 2012 508 219
- JP-A- S4 988 877
- US-A- 4 026 907
- ACKLAND M. J. ET AL: "Synthesis of carbon-14 and carbon-13 labelled (R)-(-)-2[[4-(2,6-di-1-pyrrolidinyl-4-pyrimidinyl)-1-piperazinyl]methyl]-3,4-dihydro-2,5,7,8-tetramethyl-2H-1-benzopyran-6-ol", JOURNAL OF LABELLED COMPOUNDS AND RADIOPHARMACEUTICALS, vol. 33, no. 1, 1993, GB, pages 45 - 60, XP093120336, ISSN: 0362-4803, DOI: 10.1002/jlcr.2580330107
- HIROYUKI NOHIRA: "Separation of optical isomers (quarterly chemistry review no. 6)", KIKAN-KAGAKU-SŌSETSU = QUARTERLY CHEMICAL REVIEW, no. 6, 1 January 1989 (1989-01-01), JP, pages 45 - 54, XP009530687, ISSN: 1342-5722

## Description

### [Technical Field]

The present application provides a method for chiral resolution of Trolox. The present application also provides a composition and method for optically resolving a compound, comprising an amide-based solvent.

### [Background Art]

International Publication No. WO 2009/061744 describes that racemic 2-hydroxy-2-methyl-4-(2,4,5-trimethyl-3,6-dioxocyclohexa-1,4-dienyl)butanamide, which is useful in treating and/or suppressing a mitochondrial disorder and a specific pervasive developmental disorder, is synthesized from racemic Trolox (6-hydroxy-2,5,7,8-tetramethylchroman-2-carboxylic acid).

A chiral resolving agent can be useful for separating enantiomers. For example, a chiral resolving agent can form a solid salt with one of the enantiomers, but not the other enantiomer (remains in a solution or as an oil). Accordingly, these two enantiomers can be separated by filtering a solid. However, not all resolving agents are useful for separating enantiomers of a specific compound. Furthermore, resolving agents vary, for example, in their ability to provide better resolution, higher yield, simpler scale-up, and/or improved usability.

Racemic Trolox has been resolved into (R) - and (S)-isomers by using α-methylbenzylamine (MBA) and R-(+) -N-benzyl-α-phenylethylamine resolving agents up to this point. See for example US Patent No. 3,947,473, 4,003,919, and 4,026,907, and US Patent Application Publication No. 2011/0251407.
WO2016/100579 discloses polymorphic and amorphous forms of anhydrate, hydrate, and solvates of (R)-2-hydroxy-2-methyl-4-(2,4,5-trimethyl-3,6-dioxocyclohexa-1,4-dienyl)butanamide
WO2016/100576 discloses methods of separating Trolox isomers (R)-Trolox and (S)-Trolox
EP2351729 discloses a method of separating an organic carboxylic acid salt in a mother liquor and a crystallized organic carboxylic acid from each other
WO02/12221 discloses a method for optical resolution of (+/-)-6-hydroxy-2,5,7,8-tetramethylcoumarone-2-carboxylic acid
US4026907 discloses (6-hydroxy-chroman-2-yl) acetic or carboxylic acid derivatives useful as antioxidants and a method for preparing these derivatives from hydroquinones Ackland M. J. et al: "Synthesis of carbon-14 and carbon-13 labelled (R)-(-)-2[[4-(2,6-di-1-pyrrolidinyl-4-pyrimidinyl)-1-piperazinyl]methyl]-3,4-dihydro-2,5,7,8-tetramethyl-2H-1-benzopyran-6-ol",Journal of Labelled Compounds and Radiopharmaceuticals, vol. 33, no. 1, 1993, GB, pages 45 - 60 discloses synthesis and characterization of the title compound.

### [Citation List]

### [Patent Literature]

[PTL 1] International Publication No. WO 2009/061744
[PTL 2] US Patent No. 3,947,473
[PTL 3] US Patent No. 4,003,919
[PTL 4] US Patent No. 4,026,907
[PTL 5] US Patent Application Publication No. 2011/0251407

### [Summary of Invention]

### [Solution to Problem]

The inventors have completed the present disclosure by finding a method of optically resolving Trolox stably as a result of diligent study. The present application has also found that an amide-based solvent is useful for optically resolving a compound.

For example, the present disclosure provides the following items.
(Item 1)
   A method of manufacturing a solid salt of a compound of formula I, comprising adding an amide-based solvent in the presence of an optical resolving agent to a sample comprising the compound of formula I and suspected of comprising a compound of formula II: 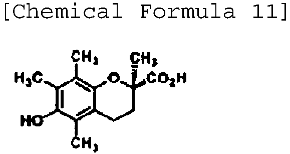
   Formula I: (R)-6-hydroxy-2,5,7,8-tetramethylchroman-2-carboxylic acid (hereinafter R Trolox) 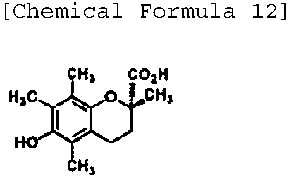
   Formula II: (S)-6-hydroxy-2,5,7,8-tetramethylchroman-2-carboxylic acid (hereinafter S Trolox)
      wherein the solid salt is an amide-based solvate,
      wherein the optical resolving agent is selected from phenylethylamine, N-methylglucamine, arginine, lysine, pseudoephedrine, leucinol, and aminophenyl propanol,wherein the amide-based solvent is one or more solvents selected from N-methylpyrrolidone (NMP), dimethylacetamide (DMA), and N,N-dimethylformamide (DMF).
(Item 2) The method of item 1, wherein the optical resolving agent comprises at least an asymmetric carbon, and a moiety that can form a salt with the R Trolox and S Trolox.
(Item 3) The method of item 1 or 2, comprising adding the optical resolving agent to the sample.
(Item 4) The method of any one of items 1 to 3, wherein addition of the amide-based solvent comprises adding a mixture of the optical resolving agent and the amide-based solvent to the sample.
(Item 5) The method of any one of items 1 to 4, wherein the sample comprises a complex with the optical resolving agent.
(Item 6) The method of any one of items 1 to 5, wherein the sample comprises a mixture of a compound of formula I and a compound of formula II.
(Item 8) The method of any one of items 1 to 6, wherein the optical resolving agent is phenylethylamine.
(Item 10) The method of item 1, wherein the amide-based solvent is N-methylpyrrolidone.
(Item 11) The method of item 1, wherein the amide-based solvent is dimethylacetamide.
(Item 12) The method of item 1, wherein the amide-based solvent is N,N-dimethylformamide.
(Item 13) The method of any one of items 1 to 12, wherein the phenylethylamine is (R)-phenylethylamine.
(Item 14) The method of any one of items 1 to 13, wherein the solid salt is an R-phenylethylamine salt.
(Item 20) The method of any one of items 1 to 14, wherein an enantiomeric excess of a manufactured solid salt of a compound of formula I is at least 98% or greater.
(Item 21) The method of any one of items 1 to 20, wherein an enantiomeric excess of a manufactured solid salt of a compound of formula I is at least 99% or greater.
(Item 22) The method of any one of items 1 to 21, further comprising dissolving the phenylethylamine into toluene.
(Item 23) The method of any one of items 1 to 22, comprising adding a poor solvent to the sample.
(Item 24) The method of item 23, wherein the poor solvent is toluene or ethyl acetate.
(Item 25) The method of any one of items 1 to 24, further comprising removing the amide-based solvent.
(Item 26) There is disclosed a method of manufacturing a compound of formula IIIa, comprising converting a solid salt of a compound of formula I manufactured by the method of any one of items 1 to 25 into a compound of formula IIIa 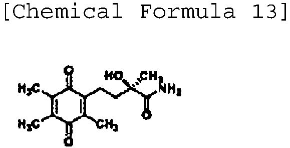 Formula IIIa: (R) -2-hydroxy-2-methyl-4- (2,4,5-trimethyl-3,6-dioxocyclohexa-1,4-dienyl)butanamide.
(Item 27) There is disclosed a compound of formula IVa: 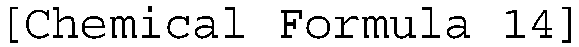 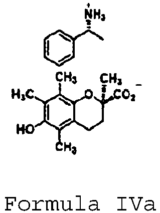 or a solvate thereof.
(Item 28) The compound of item 27, which is an amide-based solvate.
(Item 29) The solvate of any one of items 27 to 28, wherein an amide-based solvent is one or more solvents selected from NMP (N-methylpyrrolidone), DMA (dimethylacetamide), and DMF (N,N-dimethylformamide).
(Item 30) The solvate of item 29, wherein the amide-based solvent is N-methylpyrrolidone.
(Item 31) The solvate of item 29, wherein the amide-based solvent is dimethylacetamide.
(Item 32) The solvate of item 29, wherein the amide-based solvent is N,N-dimethylformamide.
(Item 33) A pharmaceutical ingredient comprising the compound or solvate of any one of items 27 to 32.
(Item 34) There is disclosed a method of manufacturing a solid salt of a compound of formula II, comprising adding an amide-based solvent in the presence of an optical resolving agent to a sample comprising a compound of formula II and suspected of comprising a compound of formula I: 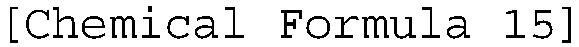 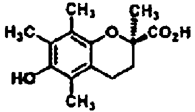
   Formula I: (R)-6-hydroxy-2,5,7,8-tetramethylchroman-2-carboxylic acid (hereinafter R Trolox) 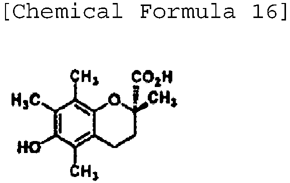
   Formula II: (S)-6-hydroxy-2,5,7,8-tetramethylchroman-2-carboxylic acid (hereinafter S Trolox).
(Item 35) The method of item 34, wherein the optical resolving agent comprises at least an asymmetric carbon, and a moiety that can form a salt with the R Trolox and S Trolox.
(Item 36) The method of item 34 or 35, comprising adding the optical resolving agent to the sample.
(Item 37) The method of any one of items 34 to 36, wherein addition of the amide-based solvent comprises adding a mixture of the optical resolving agent and the amide-based solvent to the sample.
(Item 38) The method of any one of items 34 to 37, wherein the sample comprises a complex with the optical resolving agent.
(Item 39) The method of any one of items 34 to 38, wherein the sample comprises a mixture of a compound of formula I and a compound of formula II.
(Item 40) The method of any one of items 34 to 39, wherein the optical resolving agent is selected from phenylethylamine, N-methylglucamine, arginine, lysine, pseudoephedrine, leucinol, and aminophenyl propanol.
(Item 41) The method of any one of items 34 to 40, wherein the optical resolving agent is phenylethylamine.
(Item 42) The method of any one of items 34 to 41, wherein the amide-based solvent is one or more solvents selected from N-methylpyrrolidone (NMP), dimethylacetamide (DMA), and N,N-dimethylformamide (DMF).
(Item 43) The method of item 42, wherein the amide-based solvent is N-methylpyrrolidone.
(Item 44) The method of item 42, wherein the amide-based solvent is dimethylacetamide.
(Item 45) The method of item 42, wherein the amide-based solvent is N,N-dimethylformamide.
(Item 46) The method of any one of items 40 to 45, wherein the phenylethylamine is (R)-phenylethylamine.
(Item 47) The method of any one of items 34 to 46, wherein the solid salt is an S-phenylethylamine salt.
(Item 48) The method of any one of items 34 to 47, wherein the solid salt is an amide-based solvate.
(Item 49) The method of any one of items 34 to 48, wherein the amide-based solvent is one or more solvents selected from N-methylpyrrolidone (NMP), dimethylacetamide (DMA), and N,N-dimethylformamide (DMF).
(Item 50) The method of item 49, wherein the amide-based solvent is N-methylpyrrolidone.
(Item 51) The method of item 49, wherein the amide-based solvent is dimethylacetamide.
(Item 52) The method of item 49, wherein the amide-based solvent is N,N-dimethylformamide.
(Item 53) The method of any one of items 34 to 52, wherein an enantiomeric excess of a manufactured solid salt of a compound of formula II is at least 98% or greater.
(Item 54) The method of any one of items 34 to 53, wherein an enantiomeric excess of a manufactured solid salt of a compound of formula II is at least 99% or greater.
(Item 55) The method of any one of items 34 to 54, further comprising dissolving the phenylethylamine into toluene.
(Item 56) The method of any one of items 34 to 55, comprising adding a poor solvent to the sample.
(Item 57) The method of item 56, wherein the poor solvent is toluene or ethyl acetate.
(Item 58) The method of any one of items 34 to 57, further comprising removing the amide-based solvent.
(Item 59) There is disclosed a method of manufacturing a compound of formula IIIb, comprising converting a solid salt of a compound of formula II manufactured by the method of any one of items 34 to 58 into a compound of formula IIIb 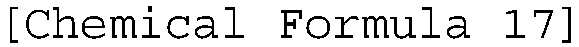 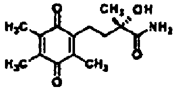 Formula IIIb: (S)-2-hydroxy-2-methyl-4-(2,4,5-trimethyl-3,6-dioxocyclohexa-1,4-dienyl)butanamide.
(Item 60) There is disclosed a compound of formula IVb: 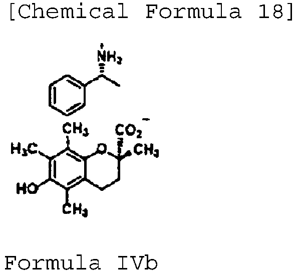 or a solvate thereof.
(Item 61) The compound of item 60, which is an amide-based solvate.
(Item 62) The solvate of any one of items 60 to 61, wherein the amide-based solvent is one or more solvents selected from NMP (N-methylpyrrolidone), DMA (dimethylacetamide), and DMF (N,N-dimethylformamide).
(Item 63) The solvate of item 62, wherein the amide-based solvent is N-methylpyrrolidone.
(Item 64) The solvate of item 62, wherein the amide-based solvent is dimethylacetamide.
(Item 65) The solvate of item 62, wherein the amide-based solvent is N,N-dimethylformamide.
(Item 66) There is disclosed a pharmaceutical ingredient comprising the compound or solvate of any one of items 60 to 65.
(Item 67) There is disclosed a composition for use in optically resolving a compound, comprising an amide-based solvent.
(Item 68) The composition of item 67, wherein the compound is a mixture of a compound of formula I and a compound of formula II: 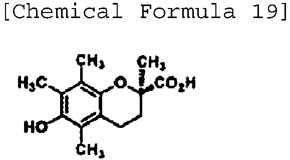
   Formula I: (R)-6-hydroxy-2,5,7,8-tetramethylchroman-2-carboxylic acid (hereinafter R Trolox) 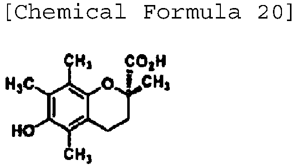
   Formula II: (S)-6-hydroxy-2,5,7,8-tetramethylchroman-2-carboxylic acid (hereinafter S Trolox).
(Item 69) The composition of item 67 or 68, wherein the composition is used with another optical resolving agent.
(Item 70) The composition of item 69, wherein the another optical resolving agent itself has optical activity.
(Item 71) The composition of any one of items 67 to 70, wherein the amide-based solvent is one or more solvents selected from N-methylpyrrolidone (NMP), dimethylacetamide (DMA), and N,N-dimethylformamide (DMF).
(Item 72) The composition of item 71, wherein the amide-based solvent is N-methylpyrrolidone.
(Item 73) The composition of item 71, wherein the amide-based solvent is dimethylacetamide.
(Item 74) The composition of item 71, wherein the amide-based solvent is N,N-dimethylformamide.
(Item 74A) The method of item 67, further comprising a feature of any one or more of items 1 to 74.
(Item 75) A method of optically resolving a compound, comprising adding an amide-based solvent to the compound.
(Item 75A) The method of item 75, further comprising a feature of any one or more of items 1 to 74.
(Item 76) Use of an amide-based solvent for optically resolving a compound.
(Item 76A) The method of item 76, further comprising a feature of any one or more of items 1 to 75.

The present disclosure is intended so that one or more of the aforementioned features can be provided not only as the explicitly disclosed combinations, but also as other combinations. Additional embodiments and advantages of the present disclosure are recognized by those skilled in the art by reading and understanding the following detailed descriptions as needed.

### [Advantageous Effects of Invention]

The present application provides a method of chirally resolving Trolox stably at a high optical purity.

### [Brief Description of Drawings]

[Figure 1] Figure 1 shows a single crystal X-ray structure of RS-Trolox•R-PEA (racemate: R-Trolox•R-PEA•S-Trolox•R-PEA).
[Figure 2] Figure 2 shows a single crystal X-ray structure of R-Trolox•R-PEA•NMP.
[Figure 3] Figure 3 shows results of X-ray powder diffraction (XRPD) on RS-Trolox•R-PEA (racemate), R-Trolox•R-PEA, and R-Trolox•R-PEA•NMP (solvate). The value at the left end of the horizontal axis of Figure 3 is "5".

### [Description of Embodiments]

The present disclosure is described hereinafter in more detail. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

The present disclosure is further described in detail hereinafter.

The abbreviations used herein have the conventional meaning within the scope of the art unless specifically noted otherwise.

The term "about" for a value or parameter herein includes variations about the value or parameter itself. Unless specifically noted otherwise, "about X" for example includes "X" itself as well as values with an acceptable error of ± 10% therefrom.

As used herein, "Trolox" indicates 6-hydroxy-2,5,7,8-tetramethylchroman-2-carboxylic acid. R form is referred to as R Trolox, and S form is referred to as S Trolox. Trolox can be prepared by a synthesis method that is well known to those skilled in the art, such as the methods described in US Patent No. 3,947,473, US Patent No. 4,003,919, and US Patent No. 4,026,907.

As used herein, "optical resolving agent" refers to an optically active reagent that is useful for separating a racemate into each of the enantiomers, i.e., optical active substances. In the present disclosure, an optical resolving agent can comprise at least an asymmetric carbon, and a moiety that can form a salt with the R Trolox and S Trolox. In the present disclosure, an optical resolving agent can comprise a base moiety. A base moiety can comprise an amine moiety. Examples of optical resolving agents include optical active substances such as phenylethylamine, N-methylglucamine, arginine, lysine, pseudoephedrine, leucinol, and aminophenyl propanol.

As used herein, "moiety that can form a salt with R Trolox and S Trolox" refers to a moiety comprising a functional group that can form a salt with a functional group comprised in R Trolox and S Trolox. For example, Trolox comprises a carboxyl group, so that the "moiety that can form a salt with R Trolox and S Trolox" can be a moiety comprising a base that can form a salt with a carboxyl group. The "moiety that can form a salt with R Trolox and S Trolox" in an optical resolving agent may or may not overlap with asymmetric carbon in the optical resolving agent. Overlapping is conditional on asymmetric carbon effectively functioning (exhibits a behavior that is different from asymmetric carbon of other steric configurations) when or after an optical resolving agent forms a salt with R Trolox and S Trolox. Examples of "moiety that can form a salt with R Trolox and S Trolox" include amino group, imino group, guanidyl group, heterocyclic group (nitrogen containing heterocyclic group such as pyridine, imidazole, indole, or purine) comprising moieties and the like. Examples of compounds comprising asymmetric carbon include tartaric acid, phenylethylamine, N-methylglucamine, amino acid (arginine, lysine, and the like), pseudoephedrine, leucinol, aminophenyl propanol, and the like. Asymmetric carbon having the same backbone as such compounds can be used.

As used herein, "amide-based solvent" refers to a solvent of a compound comprising an amide group (acid amide group). Examples of amide groups include carboxylic acid amide, phosphoric acid amide groups, and the like. Examples of amide-based solvents include N-methylpyrrolidone (NMP), dimethylacetamide (DMA), N,N-dimethylformamide (DMF), N,N-diethylformamide (DEF), tetramethylurea (TMU), and hexamethylphosphoric triamide (HMPA).

As used herein, "poor solvent" refers to a solvent that has the ability to dissolve a solute (substance of interest), but has limited solubility (solvent with low capability to dissolve a solute). Examples of poor solvents in the present disclosure include ethyl acetate, toluene, and the like.

As used herein, "salt" indicates a compound generated by a neutralization reaction between an acid and a base, consisting of a negative component of the acid and positive component of the base. Trolox has a carboxylic acid group and can form a salt with a basic substance.

As used herein, "solid salt" indicates a salt in a solid state among the three states of matter (gas/liquid/solid).

As used herein, "suspect" indicates that something is not confirmed, but is sufficiently expected. This can be false after actual confirmation. For example, "sample comprising the compound of formula I and suspected of comprising a compound of formula II" refers to a sample that comprises a compound of formula I while not confirmed to comprise a compound of formula II, but is sufficiently expected to comprise a compound of formula II.

As used herein, "mixture" indicates two or more substances that are mixed. The ratio of mixture can be any ratio. For example, for a mixture of two substances, the ratio can be 50:50, 45:55, 40:60, 35:65, 30:70, 25:75, 20:80, 15:85, 10:90, 5:95, 1:99, 0.1:99.9, or the like.

As used herein, "enantiomeric excess" indicates the scale representing the ratio of right enantiomer to left enantiomer in a mixture and is abbreviated as ee. If the ratios of the right and left enantiomers in a sample are R and S, respectively, ee can be found by using the following equation. $ee \left(%\right) = \left(R-S\right) / \left(R+S\right) \times 100 , or \left(S-R\right) / \left(R+S\right) \times 100$ For example, the enantiomeric excess of R form is $\left(90-10\right) / \left(90+10\right) \times 100 = 80 % ee$ at a ratio of R:S = 90:10.

### (Preferred embodiments)

One embodiment of the present disclosure provides a method of manufacturing a solid salt of a compound of formula I, comprising adding an amide-based solvent in the presence of an optical resolving agent to a sample comprising the compound of formula I and suspected of comprising a compound of formula II: 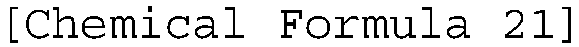 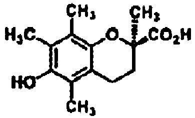
Formula I: (R)-6-hydroxy-2,5,7,8-tetramethylchroman-2-carboxylic acid (hereinafter R Trolox) 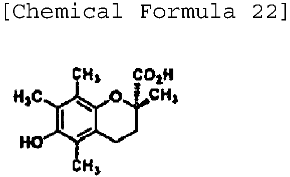
Formula II: (S)-6-hydroxy-2,5,7,8-tetramethylchroman-2-carboxylic acid (hereinafter S Trolox). R Trolox can be optically resolved stably with the method of the present disclosure. This method provides a solid that can be more readily scaled-up, more readily stirred, and readily filtered, and provides better resolution and/or purity for a desired product.

A solid salt of a compound of formula I is a salt of a compound of formula I and an optical resolving agent. A solid salt of a compound of formula I is a solvate of a salt of a compound of formula I and an optical resolving agent and an amide-based solvent.

In this disclosure, the optical resolving agent can comprise at least an asymmetric carbon, and a moiety that can form a salt with the R Trolox and S Trolox. Since Trolox comprises a carboxylic acid moiety, an optical resolving agent can comprise a base moiety forming a salt with carboxylic acid. A base moiety can be amine. For example, if an optical resolving agent is R-phenylethylamine, R Trolox and R-phenylethylamine can form a salt such as the following 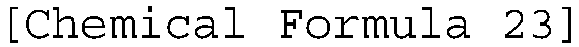 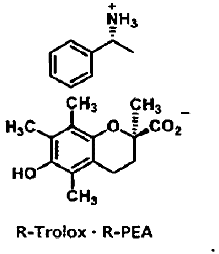 If a salt of R Trolox and R-phenylethylamine is solvated with N-methylpyrrolidone, this can result in a solvate such as the following 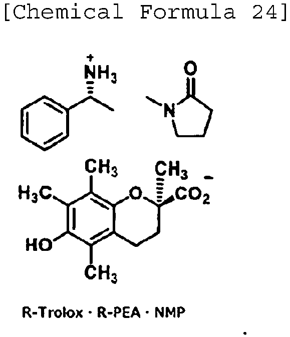 If an optical resolving agent comprises an amine moiety of N-methylglucamine, arginine, lysine, pseudoephedrine, leucinol, aminophenyl propanol, or the like, a salt with the amine moiety that is ammonium can be formed.

The method of the present disclosure comprises adding an optical resolving agent to the sample.

The addition of the amide-based solvent can be achieved by adding a mixture of the optical resolving agent and the amide-based solvent to the sample. When adding an amide-based solvent, an optical resolving agent and/or amide-based solvent can be already present in a sample (thus, a new amide-based solvent may or may not be added). Addition of an optical resolving agent and addition of an amide-based solvent can be concurrent or independent.

In the present disclosure, the sample can comprise a complex with the optical resolving agent.

In the present disclosure, the sample can comprise a mixture of a compound of formula I and a compound of formula II.

In the present disclosure, the optical resolving agent is selected from phenylethylamine, N-methylglucamine, arginine, lysine, pseudoephedrine, leucinol, and aminophenyl propanol. An optical resolving agent can be R-phenylethylamine, S-phenylethylamine, N-methyl-D-glucamine, N-methyl-L-glucamine, D-arginine, L-arginine, D-lysine, **L-**lysine, (1S,2S)-(+)-pseudoephedrine, (1R,2R)-(-)-pseudoephedrine, (R)-(-)-leucinol, (S)-(+)-leucinol, (S)-(-)-2-amino-3-phenyl-1-propanol, or (R)-(+)-2-amino-3-phenyl-1-propanol.

In the present disclosure, the optical resolving agent can be phenylethylamine.

In the present disclosure, the amide-based solvent can be one or more solvents selected from N-methylpyrrolidone (NMP), dimethylacetamide (DMA), and N,N-dimethylformamide (DMF).

In the present disclosure, the amide-base solvent can be N-methylpyrrolidone.

In the present disclosure, the amide-base solvent can be dimethylacetamide.

In the present disclosure, the amide-base solvent can be N,N-dimethylformamide.

In the present disclosure, the phenylethylamine can be (R)-phenylethylamine.

In the present disclosure, the solid salt can be an R-phenylethylamine salt.

In the present disclosure, the solid salt can be an amide-based solvate.

In the present disclosure, the amide-based solvent can be one or more solvents selected from N-methylpyrrolidone (NMP), dimethylacetamide (DMA), and N,N-dimethylformamide (DMF).

In the present disclosure, the amide-based solvent can be N-methylpyrrolidone.

In the present disclosure, the amide-based solvent can be dimethylacetamide.

In the present disclosure, the amide-based solvent can be N,N-dimethylformamide.

In the present disclosure, an enantiomeric excess of a manufactured solid salt of a compound of formula I can be at least 98% or greater.

In the present disclosure, an enantiomeric excess of a manufactured solid salt of a compound of formula I can be at least 99% or greater.

In the present disclosure, the method can further comprise dissolving the phenylethylamine into toluene.

In the present disclosure, the method can comprise adding a poor solvent to the sample. Advantages of using a poor solvent include improved filterability. While addition of a poor solvent can reduce the viscosity of an amide-based solvent to improve the practicality, the present disclosure is not limited thereto. The optical purity can also be slightly improved by adding a poor solvent.

In the present disclosure, the poor solvent can be toluene or ethyl acetate.

In the present disclosure, the method can further comprise removing the amide-based solvent.

The present disclosure provides a method of manufacturing a compound of formula IIIa, comprising converting a solid salt of a compound of formula I manufactured by the method described above into a compound of formula IIIa: 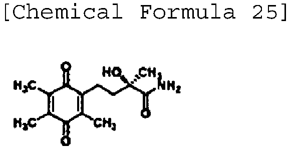 Formula IIIa: (R)-2-hydroxy-2-methyl-4- (2,4,5-trimethyl-3,6-dioxocyclohexa-1,4-dienyl)butanamide.

The present disclosure provides a compound of formula IVa: 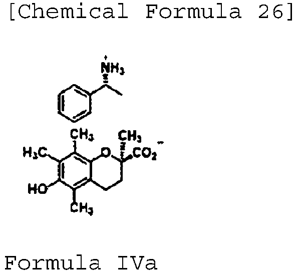 or a solvate thereof.

In the present disclosure, the compound can be an amide-based solvate.

In the present disclosure, the amide-based solvent can be one or more solvents selected from NMP (N-methylpyrrolidone), DMA (dimethylacetamide), and DMF (N,N-dimethylformamide).

In the present disclosure, the amide-based solvent can be N-methylpyrrolidone.

In the present disclosure, the amide-based solvent can be dimethylacetamide.

In the present disclosure, the amide-based solvent can be N,N-dimethylformamide.

In the present disclosure, a pharmaceutical ingredient comprising a compound or solvate described above can be provided.

The present disclosure provides a method of manufacturing a solid salt of a compound of formula II, comprising adding an amide-based solvent in the presence of an optical resolving agent to a sample comprising a compound of formula II and suspected of comprising a compound of formula I:

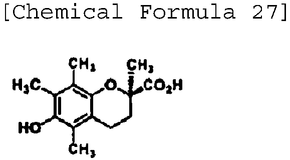

Formula I: (R)-6-hydroxy-2,5,7,8-tetramethylchroman-2-carboxylic acid (hereinafter R Trolox) 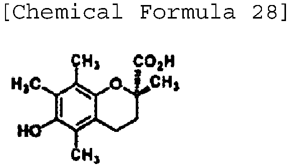 Formula II: (S)-6-hydroxy-2,5,7,8-tetramethylchroman-2-carboxylic acid (hereinafter S Trolox). Trolox can be optically resolved stably by the method of the present disclosure. This method provides a solid that can be more readily scaled-up, more readily stirred, and readily filtered and provides better resolution and/or purity of a desired product.

A solid salt of a compound of formula II can be a salt of the compound of formula II and an optical resolving agent. A solid salt of a compound of formula II can be a solvate of a salt of the compound of formula II and an optical resolving agent and an amide-based solvent.

In the present disclosure, the optical resolving agent can comprise at least an asymmetric carbon, and a moiety that can form a salt with the R Trolox and S Trolox. Since Trolox comprises a carboxylic acid moiety, an optical resolving agent can comprise a base moiety forming a salt with carboxylic acid. A base moiety can be amine. For example, if an optical resolving agent is S-phenylethylamine, S Trolox and S-phenylethylamine can form a salt in an enantiomeric relationship with the R form described above.

The method of the present disclosure can comprise adding the optical resolving agent to the sample.

The addition of the amide-based solvent can be achieved by adding a mixture of the optical resolving agent and the amide-based solvent to the sample. When adding an amide-based solvent, an optical resolving agent and/or amide-based solvent can be already present in a sample (thus, a new amide-based solvent may or may not be added). Addition of an optical resolving agent and addition of an amide-based solvent can be concurrent or independent.

In the present disclosure, the sample can comprise a complex with the optical resolving agent.

In the present disclosure, the sample can comprise a mixture of a compound of formula I and a compound of formula II.

In the present disclosure, the optical resolving agent can be selected from phenylethylamine, N-methylglucamine, arginine, lysine, pseudoephedrine, leucinol, and aminophenyl propanol. An optical resolving agent can be R-phenylethylamine, S-phenylethylamine, N-methyl-D-glucamine, N-methyl-L-glucamine, D-arginine, L-arginine, D-lysine, **L-**lysine, (1S,2S)-(+)-pseudoephedrine, (1R,2R)-(-)-pseudoephedrine, (R)-(-)-leucinol, (S)-(+)-leucinol, (S)-(-)-2-amino-3-phenyl-1-propanol, or (R)-(+)-2-amino-3-phenyl-1-propanol.

In the present disclosure, the optical resolving agent can be phenylethylamine.

In the present disclosure, the amide-based solvent can be one or more solvents selected from N-methylpyrrolidone (NMP), dimethylacetamide (DMA), and N,N-dimethylformamide (DMF).

In the present disclosure, the amide-based solvent can be N-methylpyrrolidone.

In the present disclosure, the amide-based solvent can be dimethylacetamide.

In the present disclosure, the amide-based solvent can be N,N-dimethylformamide.

In the present disclosure, the phenylethylamine can be (R)-phenylethylamine.

In the present disclosure, the solid salt can be an S-phenylethylamine salt.

In the present disclosure, the solid salt can be an amide-based solvate.

In the present disclosure, the amide-based solvent can be one or more solvents selected from N-methylpyrrolidone (NMP), dimethylacetamide (DMA), and N,N-dimethylformamide (DMF).

In the present disclosure, the amide-based solvent can be N-methylpyrrolidone.

In the present disclosure, the amide-based solvent can be dimethylacetamide.

In the present disclosure, the amide-based solvent can be N,N-dimethylformamide.

In the present disclosure, an enantiomeric excess of a manufactured solid salt of a compound of formula II can be at least 98% or greater.

In the present disclosure, an enantiomeric excess of a manufactured solid salt of a compound of formula II can be at least 99% or greater.

In the present disclosure, the method can further comprise dissolving the phenylethylamine into toluene.

In the present disclosure, the method can comprise adding a poor solvent to the sample. Advantages of using a poor solvent include improved filterability. While addition of a poor solvent can reduce the viscosity of an amide-based solvent to improve the practicality, the present disclosure is not limited thereto. The optical purity can also be slightly improved by adding a poor solvent.

In the present disclosure, the poor solvent can be toluene or ethyl acetate.

In the present disclosure, the method can further comprise removing the amide-based solvent.

The present disclosure provides a method of manufacturing a compound of formula IIIb, comprising converting a solid salt of a compound of formula II manufactured by the method described above into a compound of formula IIIb: 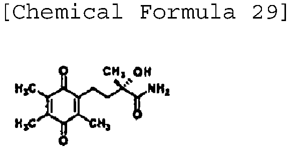 Formula IIIb: (S)-2-hydroxy-2-methyl-4-(2,4,5-trimethyl-3,6-dioxocyclohexa-1,4-dienyl)butanamide.

One embodiment of the present disclosure provides a compound of formula IVb: 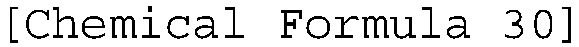 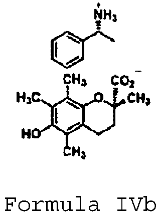 or a solvate thereof.

In the present disclosure, the compound can be an amide-based solvate.

In the present disclosure, the amide-based solvent can be one or more solvents selected from NMP (N-methylpyrrolidone), DMA (dimethylacetamide), and DMF (N,N-dimethylformamide).

In the present disclosure, the amide-based solvent can be N-methylpyrrolidone.

In the present disclosure, the amide-based solvent can be dimethylacetamide.

In the present disclosure, the amide-based solvent can be N,N-dimethylformamide.

The present disclosure can provide a pharmaceutical ingredient comprising a compound or solvate described above.

One embodiment of the present disclosure provides a composition for use in optically resolving a compound, comprising an amide-based solvent.

In the present disclosure, the compound can be a mixture of a compound of formula I and a compound of formula II: 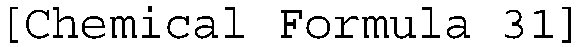 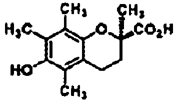 Formula I: (R)-6-hydroxy-2,5,7,8-tetramethylchroman-2-carboxylic acid (hereinafter R Trolox) 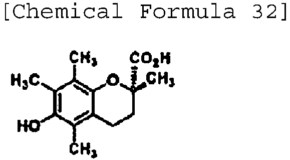 Formula II: (S)-6-hydroxy-2,5,7,8-tetramethylchroman-2-carboxylic acid (hereinafter S Trolox).

In the present disclosure, the composition can be used with another optical resolving agent.

In the present disclosure, the another optical resolving agent itself can have optical activity.

In the present disclosure, the amide-based solvent can be one or more solvents selected from N-methylpyrrolidone (NMP), dimethylacetamide (DMA), and N,N-dimethylformamide (DMF).

In the present disclosure, the amide-based solvent can be N-methylpyrrolidone.

In the present disclosure, the amide-based solvent can be dimethylacetamide.

In the present disclosure, the amide-based solvent can be N,N-dimethylformamide.

One embodiment of the present disclosure provides a method of optically resolving a compound, comprising adding an amide-based solvent to the compound.

One embodiment of the present disclosure provides use of an amide-based solvent for optically resolving a compound.

In one embodiment of the present disclosure, X-ray powder diffraction (XRPD) on RS-Trolox•R-PEA (racemate) exhibits 2θ peaks at 8.54°, 12.55°, 13.45°, 15.41°, 17.11°, 18.87°, 21.00°, 22.19°, and 23.83°, but the peaks are not limited thereto. X-ray powder diffraction may have an appropriately number of peaks (1, 2, 3, 4, 5, 6, 7, 8, 9, 10 or any integer value that is greater) selected from angles with the largest Gross Intensity specified in Table 6. In regards to the assignment of X-ray powder diffraction (XRPD), 2θ can have an error in the range of ± 0.2° with respect to the exhibited value in the present disclosure.

In one embodiment of the present disclosure, X-ray powder diffraction (XRPD) on R-Trolox•R-PEA exhibits 2θ peaks at 6.75°, 7.19°, 9.86°, 13.49°, 14.37°, 14.67°, 15.29°, 21.51°, 22.71°, 23.31°, 27.10°, and 28.03°, but the peaks are not limited thereto. X-ray powder diffraction may have an appropriately number of peaks (1, 2, 3, 4, 5, 6, 7, 8, 9, 10 or any integer value that is greater) selected from angles with the largest Gross Intensity specified in Table 6.

In one embodiment of the present disclosure, X-ray powder diffraction (XRPD) on R-Trolox•R-PEA•NMP (solvate) exhibits 2θ peaks at 5.30°, 7.69°, 8.94°, 10.55°, 12.78°, 14.41°, 15.04°, 15.35°, 15.83°, 17.86°, 19.56°, 22.34°, 25.43°, 26.48°, and 27.47° as characteristic 2θ therein, but the peaks are not limited thereto. X-ray powder diffraction may have an appropriately number of peaks (1, 2, 3, 4, 5, 6, 7, 8, 9, 10 or any integer value that is greater) selected from angles with the largest Gross Intensity specified in Table 6.

A crystal exhibits various structures, shapes, aggregation states, and the like depending on the crystallization conditions. A solvate with a solvent incorporated in a crystal is also referred to as a crystal pseudopolymorphism to distinguish from crystal polymorphisms in the strict sense. In such a case, physicochemical properties of a crystal can be significantly influenced by the solvent's contribution to crystal lattice formation or interaction with a molecule to be crystallized. Various factors such as the properties of a solvent, degree of supersaturation, and temperature associated with crystallization conditions can determine the properties of a crystal. The present disclosure is capable of stable chiral resolution of Trolox at a high optical purity by suitably selecting an optical resolving agent and solvent.

While it was observed that Trolox could no longer be optically resolved if phenethylamine was used as an optical resolving agent in a non-amide-based solvent, Trolox was able to be optically resolved stably by using an amide-based solvent.

The method of the present disclosure utilizes an optical resolving agent for separating (R) - and (S) -Trolox enantiomers. The resolving agent forms a solid salt with one of (R)- and (S)-Trolox enantiomers, while substantially not forming a solid salt with the other under a specific reaction condition. In some embodiments, if a resolving agent forms a solid salt with a Trolox enantiomer, at least about 50% of the Trolox enantiomer forms a solid salt with the resolving agent under a specific reaction condition. In various embodiments, if a resolving agent forms a solid salt with a Trolox enantiomer, at least about 60%, at least about 70%, at least about 80%, at least about 85%, at least about 90%, at least about 95%, at least about 96%, at least about 97%, at least about 98%, or at least about 99% of the Trolox enantiomer forms a solid salt with the resolving agent under a specific reaction condition. "Substantially not forming a solid salt" indicates that less than about 10% of (non-solid salt forming) Trolox enantiomer form a solid salt with a resolving agent under a specific reaction condition. In various embodiments, "substantially not forming a solid salt" indicates that less than about 9%, less than about 8%, less than about 7%, less than about 6%, less than about 5%, less than about 4%, less than about 3%, less than about 2%, less than about 1%, less than about 0.5%, or less than about 0.1% of (non-solid salt forming) Trolox enantiomer form a solid salt with a resolving agent under a specific reaction condition. A solid salt can form a solvate with an amide-based solvent.

"Yield" indicates the % of Trolox enantiomer obtained relative to the amount of starting material. For example, if 100 g of 50/50 racemic mixture of (R)/(S)-Trolox is resolved and 50 g of (S)-Trolox is recovered, the yield is 50%. If 30 g of (S)-Trolox is recovered, the yield is 30%. For recovery of Trolox salt, the yield is not calculated as salt counterion, but by assuming only Trolox is present. For example, if 100 g of 50/50 racemic mixture of (R)/(S)-Trolox is resolved, 40 g of (S)-Trolox salt is recovered, and the theoretical weight of (S)-Trolox contained in the salt is 30 g, the yield is 30%. If (R)-Trolox and (S)-Trolox are both resolved and 50 g of each is recovered, the yield is 100%.

While the compounds described herein can be produced and used as a neutral (non-salt) compound, the descriptions are intended to encompass all salts of the compounds described herein and a method using such salts of compounds. In one embodiment, a salt of a compound includes a pharmaceutically acceptable salt. A pharmaceutically acceptable salt is a salt that can be administered as a drug or a pharmaceutical product to humans and/or animals and retains at least some of the biological activity of a free compound (neutral compound or non-salt compound) upon administration. A desirable salt of a basic compound can be prepared using a method known to those skilled in the art by treating the compound with an acid. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acid, and salicylic acid. Salts of basic compounds with amino acid such as aspartate and glutamate can also be prepared. A desired salt of an acidic compound can be prepared by a method known to those skilled in the art by treating the compound with a base. Examples of inorganic salts of an acid compound include, but are not limited to, alkali metals and alkaline earth salts, such as sodium salt, potassium salt, magnesium salt, and calcium salt; ammonium salts; and aluminum salts. Examples of organic salts of an acid compound include, but are not limited to, salts of procaine, dibenzylamine, N-ethylpiperidine, N,N-dibenzylethylenediamine, and triethylamine. Salts of an acidic compound with amino acid such as lysine salt or the like can also be prepared. Additional salts that are particularly useful for pharmaceutical preparations are described in Berge S.M. et al., "Pharmaceutical salts", 1. Pharm. Sci., January 1977; Vol. 66 (No. 1): pages 1 to 19.

### Applications

Compounds manufactured by the method of the present disclosure are useful as a pharmaceutical ingredient. The compounds can be used as an intermediate for preparing an optically active medicament.

### Synthesis reaction parameter

Examples of solvents utilized in the synthesis of the compounds and compositions of the present disclosure include water, acetonitrile ("ACN"), diethyl ether, 2-methyltetrahydrofuran ("2-MeTHF") , ethyl acetate ("EtOAc"), ethanol ("EtOH"), isopropyl alcohol ("IPA"), isopropyl acetate ("IPAc"), methanol (MeOH), and the like, and mixtures thereof.

The term "q.s. (appropriate amount)" means to add an amount sufficient to achieve the described function, e.g., to reach a desired volume (e.g., 100%) of a solution.

The technologies that are useful for synthesizing the compounds and compositions herein are obvious and readily available to those skilled in the art by considering the teachings described herein. The following discussion is provided for exemplification of a specific method among the various methods that are available for use in the construction of the compounds and compositions herein. However, the discussion is not intended to define the scope of the reaction that is useful in preparing the compounds and compositions herein or the order of reactions.

Other methods for generating the compounds and compositions of the present disclosure are apparent to those skilled in the art by reviewing the teachings herein.

### [Examples]

The following abbreviations can also be used in the Examples and the Tables therein to simplify the descriptions of the specification.
Trolox: 6-hydroxy-2,5,7,8-tetramethylchroman-2-carboxylic acid
R-Trolox: R-6-hydroxy-2,5,7,8-tetramethylchroman-2-carboxylic acid
S-Trolox: S-6-hydroxy-2,5,7,8-tetramethylchroman-2-carboxylic acid
RS-Trolox: RS-6-hydroxy-2,5,7,8-tetramethylchroman-2-carboxylic acid (racemate)
PEA: 1-phenylethylamine
R-PEA: R-1-phenylethylamine
S-PEA: S-1-phenylethylamine
EtOAc: ethyl acetate
*ⁱ*PrOAc: isopropyl acetate
NMP: N-methylpyrrolidone
DMA: dimethylacetamide
DMF: N,N-dimethylformamide
DMP: N,N-dimethylpropionamide
MTBE: methyl tert-butyl ether
DME: 1,2-dimethoxyethane
2-MeTHF: 2-methyltetrahydrofuran
MEK: methyl ethyl ketone
DMSO: dimethyl sulfoxide
CDCl₃: deuterated chloroform
MeOH: methanol
MeCN: acetonitrile
IPA: 2-propanol
CDI: carbonyldiimidazole
For symbols used in NMR, δ means chemical shift, s means singlet, d means doublet, t means triplet, q means quartet, m means multiplet, and J means spin coupling constant.

S-Trolox used in the Examples was purchased from Sigma-Aldrich. RS-Trolox used in the Examples was purchased from D-STONE. A method of manufacturing a compound in the present disclosure by using a seed crystal, when the compound is made for the first time, can be carried out by utilizing the small amount of the product produced without using a seed crystal as a seed crystal for the next run.

### (Method of measuring optical purity)

Optical purity is measured and calculated by comparing each peak area by high performance liquid chromatography (HPLC). The measurement conditions are the following. Retention time is indicated as Rt (min).

### Optical purity

Column: Chiralpak AD-3 3 µm (250 × 4.6 mm)
Mobile phase: hexane/2-propanol-trifluoroacetate: 900/100/1 Flow rate: 1.0 mL/min
Column temperature: 35°C
Wavelength: 205 nm

The Rt of each compound measured under the above measurement conditions is shown in the following Table.

**[Table 1]**

| Rt | Compound |
|---|---|
| 11.3 | R-Trolox |
| 13.4 | S-Trolox |

### (Example 1)

### Study on solvents (non-amide-based)

S-Trolox (0.67 g), R-PEA (0.11 g), and solvent were added to R-Trolox•R-PEA (1.0 g) to reslurry the mixture at room temperature. After maintaining the temperature at 30°C for 3 hours, the slurry was cooled to 5°C and stirred overnight. After stirring overnight at 5°C, crystals were filtered to measure the optical purity of the crystals.

**[Table 2]**

| Solvent | Optical purity | Amount of product produced |
|---|---|---|
| EtOAc | -0.2%ee | 1.18g |
| 2wt% H₂O/EtOAc | -0.2%ee | 1.10g |
| IPAc | -1.0%ee | 1.27g |
| MEK | -0.1%ee | 1.07g |
| MeCN | -0. 9%ee | 1.33g |
| IPA | 94.7%ee | 0.72g |
| 2-MeTHF | 98.3%ee | 0.55g |
| DME | 80.1%ee | 0.60g |
| CPME | 66.1%ee | 0.74g |
| Toluene | 36.0%ee | 1.07g |
| Xylene | 27.3%ee | 1.23g |
| Chlorobenzene | 99.2%ee | 0.53g |

While it has been shown that the non-amide-based solvent 2-MeTHF is capable of optically resolving the compound, such solvents was not optically resolving the compound thereafter. Although optical purity was higher with 2-MeTHF and chlorobenzene , the raw material R-Trolox•R-PEA (1.0 g) is reduced to about half (0.55 g and 0.53 g, respectively).

### (Example 2)

### Study on amide-based solvent (NMP or DMA)

At room temperature, NMP (70.0 g) or DMA (70.0 g) and R-PEA were added to Trolox (10.0 g), and the temperature was raised to 50°C. The mixture was filtrated for removing debris and washed with NMP (20.0 g). After the solution was cooled to 25°C, and R-Trolox•R-PEA (0.01 g) seed crystal was seeded and incubated for 3 hours, and cooled to 10°C, incubated overnight, and then the precipitate was filtered out. The precipitate was washed with NMP or DMA (15.0 g), washed twice with IPAc (15.0 g), and then dried to obtain an amide solvate of R-Trolox•R-PEA.

**[Table 3]**

| Solvent | NMP | DMA |
|---|---|---|
| Yield | 34.3% | 27.8% |
| Optical purity | 98.3%ee | 98.6%ee |
| Solvent content | 21.3% | 16.7% |

### (Example 3)

### Method of obtaining R-Trolox•R-PEA•NMP (solvate) from RS-Trolox•R-PEA (racemate) (NMP/DMA mixture solvent)

At room temperature, NMP (10.0 g), DMA (10.0 g), and RS-Trolox (2.00 g) were added, and the temperature was raised to 50°C. The solution was then cooled to 25°C, and R-Trolox•R-PEA (0.01 g) seed crystal was seeded and incubated for 1 hour, cooled to 10°C, and incubated overnight, and then the precipitate was filtered out. The precipitate was washed with an NMP/DMA mixture solvent (1.5 g/1.5 g), washed twice with IPAc (3.0 g), and then dried to obtain an NMP solvate of R-Trolox•R-PEA (yield: 0.76 g, yield rate 30.1%, optical purity: 98.3%ee).

### (Example 4)

### Method of obtaining R-Trolox•R-PEA•NMP (solvate) from RS-Trolox•R-PEA (racemate) (NMP solvent)

RS-Trolox•R-PEA (100 mg) was dissolved in NMP (1 mL) and stirred for 3 hours. The precipitated crystals were then filtered out (optical purity: 98.7%ee)

### (Example 5)

### Solubility of RS-Trolox•R-PEA, R-Trolox•R-PEA, and S-Trolox•R-PEA to ethyl acetate solvent

Each crystal was reslurried by adding ethyl acetate. The solubility was calculated from analyzing the content of supernatant solution of each reaction solution at each temperature.

**[Table 4]**

| **Temperature [°C]** | **rac-Trolox** | **R-Trolox** | **S-Trolox** |
|---|---|---|---|
| 0 | 0.02% | 0.07% | 2.6% |
| 15 | 0.02% | 0.09% | 9.9% |
| 30 | 0.07% | 0.10% | 14.2% |
| 45 | 0.38% | 0.40% | |
| 60 | 1.08% | 0.78% | |
| 75 | 2.82% | 0.83% | |

### (Example 6)

### Solubility of R-Trolox•R-PEA, S-Trolox•R-PEA, and S-Trolox to NMP and DMA

Each crystal was reslurried by adding an amide-based solvent (NMP, DMA). The solubility was calculated from analyzing the content of supernatant solution of each reaction solution at each temperature.

**[Table 5]**

| **Temperature [°C]** | **Solubility** [%] | | | | | |
|---|---|---|---|---|---|---|
| | R-TroloxPEA | | S-TroloxPEA | | S-Trolox (Free) | |
| | NMP | DMA | NMP | DMA | NMP | DMA |
| 20 | 0.5 | 0.4 | ≧10% | | ≧10% | |

### (Example 7)

Double crystallization using NMP solvent for improving optical purity (first step: optical resolution, second step: recrystallization)

At room temperature, NMP (490.4 g) and R-PEA (28.90 g) were added to Trolox (70.0 g), and the temperature was raised to 50°C. The mixture was filtrated for removing debris and washed with NMP (140.59 g). After the solution was cooled to 25°C, R-Trolox•R-PEA (0.693 g) seed crystal was seeded and incubated for 3 hours, cooled to 10°C, and incubated overnight, and then the precipitate was filtered out. The precipitate was washed with NMP (105.0 g), washed twice with IPAc (105.0 g), and then dried to obtain an amide solvate of R-Trolox•R-PEA (yield: 43.57 g, percentage yield 33.1%, optical purity: 97.2%ee).

NMP (47.87 g) was added to the crystal obtained above (10 g), and the temperature was raised to 60°C. After the solution was cooled to 40°C, R-Trolox•R-PEA (0.1 g) seed crystal was seeded and incubated for 2 hours, cooled to 10°C, and incubated for 1.5 hours, and then the precipitate was filtered out. The precipitate was washed with NMP (16 g), washed twice with isopropyl acetate (8 g), and then dried to obtain an amide solvate of R-Trolox•R-PEA (yield: 7.61 g, percentage yield 76.1%, optical purity: 99.8%ee).

### (Example 8)

Double crystallization using NMP/toluene mixture solvent for improving optical purity and filterability (first step: optical resolution, second step: recrystallization)

At room temperature, NMP (1440 g) and toluene (5760 g) were added to Trolox (800 g), and the temperature was raised to 50°C. The mixture was filtrated for removing debris and washed with NMP/toluene mixture solvent (160 g/640 g). After adding R-PEA (232 g) at 50°C and cooling the solution to 45°C, R-Trolox•R-PEA (0.8 g) seed crystal was seeded and incubated for 2 hours. R-PEA (97 g) was added dropwise over an hour and then incubated for 30 minutes. The mixture was cooled to 0°C and incubated overnight, and then the precipitate was filtered out. The precipitate was washed twice with NMP/toluene mixture solution (320 g/1280 g), washed with toluene (1600 g), and then dried to obtain an amide solvate of R-Trolox•R-PEA (yield: 624 g, percentage yield 41.5%, optical purity: 96.8%ee).

NMP (1650 g) and toluene (4950 g) were added to the crystal obtained above (550 g), and the temperature was raised to 70°C. After 30 minutes of incubation, the solution was cooled to 60°C. R-Trolox•R-PEA (0.6 g) seed crystal was seeded and incubated for 30 minutes, cooled to 0°C, and incubated overnight, and then the precipitate was filtered out. The precipitate was washed with an NMP/toluene mixture solvent (206 g/619 g), washed with toluene (825 g), and then dried to obtain an amide solvate of R-Trolox•R-PEA (yield: 514 g, percentage yield 93.4%, optical purity: > 99.9%ee).

### (Example 9)

Double crystallization using NMP/ethyl acetate mixture solvent for improving optical purity and filterability (first step: optical resolution, second step: recrystallization)

At room temperature, NMP (10.80 kg) and ethyl acetate (43.20 kg) were added to Trolox (6.00 kg), and the temperature was raised to 50°C. After filtration to remove debris, the mixture was washed with an NMP/ethyl acetate mixture solvent (1.20 kg/4.80 kg). At 50°C, R-PEA (1.74 kg) was added and then R-Trolox•R-PEA•NMP (6.04 g) was seeded as a seed crystal and incubated for 2 hour. After adding R-PEA (0.73 kg) dropwise over 1 hour, the mixture was incubated for 30 minutes. After cooling the mixture to 5°C and incubating overnight, the precipitate was filtered out. The precipitate was washed twice with an NMP/ethyl acetate mixture solvent (2.40 kg/9.60 kg), washed twice with toluene (12.00 kg), and then dried to obtain an NMP solvate of R-Trolox•R-PEA (yield: 4.53 kg, percentage yield 40.2%, optical purity: 96.6%ee).

NMP (9.00 kg) and ethyl acetate (27.00 kg) were added to the crystal obtained above (3.00 kg), and the temperature was raised to 68°C. After the solution was cooled to 60°C, R-Trolox•R-PEA•NMP (3.02 g) was seeded as a seed crystal and incubated for 1 hour, cooled to 5°C, and incubated overnight, and then the precipitate was filtered out. The precipitate was washed twice with an NMP/ethyl acetate mixture solvent (1.13 kg/3.38 kg), washed twice with ethyl acetate (4.50 kg), and then dried to obtain an NMP solvate of R-Trolox•R-PEA (yield: 2.71 g, percentage yield 90.3%, optical purity: > 99.9%ee).

### (Example 10)

### Single crystal X ray structure analysis of RS-Trolox•R-PEA (racemate) and R-Trolox•R-PEA•NMP (solvate)

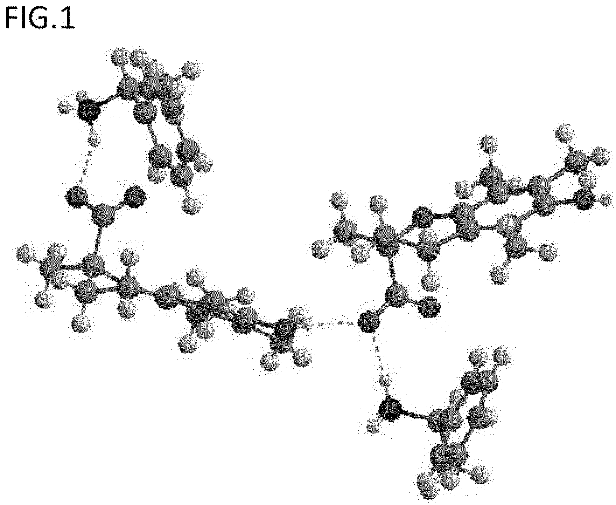

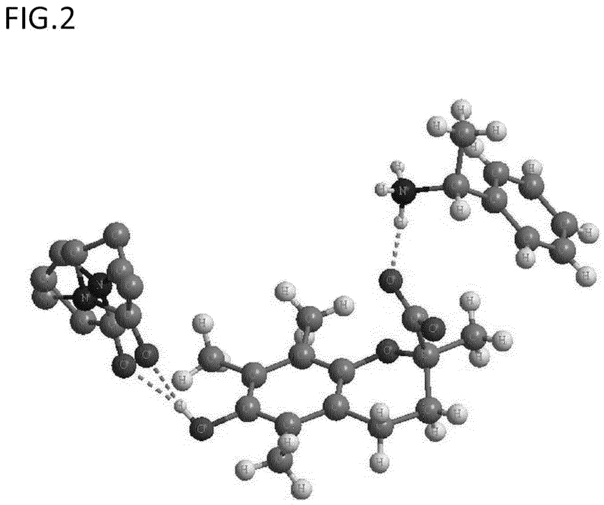

The single crystal X-ray structures of RS-Trolox•R-PEA (racemate) and R-Trolox•R-PEA•NMP (solvate) were analyzed. Figures 1 and 2 show the analysis. Measurement apparatus: Rigaku single crystal X-ray diffractometer (model number: VariMax RAPID RA-Micro7). Measurement temperature: -170°C. Analysis program: CrystalStructure crystallographic software package.

RS-Trolox•R-PEA (racemate) was specifically measured as follows.

### (Data collection)

A colorless prism crystal of C₂₂H₂₉NO₄ with approximate dimensions of 0.200 x 0.110 x 0.090 mm was placed on a glass fiber. All measurements were taken with a Rigaku R-AXIS RAPID diffractometer using multilayer mirror monochromator Mo-Kα radiation.

The distance between the crystal and detector was 127.40 mm.

The cell constants and rotation matrix for data collection were compatible with a simple triclinic unit cell having a dimension: $\begin{matrix}a = 9.04561 \left(19\right) A & α = 91.615 \left(7\right) ° \\ b = 10.3133 \left(2\right) A & β = 93.663 \left(7\right) ° \\ c = 10.3424 \left(2\right) A & γ = 94.236 \left(7\right) ° \\ v = 959.72 \left(4\right) {A}^{3} & \end{matrix}$

For Z =2 and formula weight = 371.48, the calculated density is 1.285 g/cm³. The space group was determined as P1 (#1) based on statistical analysis of the intensity distribution and successful analysis and refinement of the structure.

Data was collected up to the maximum 2θ value of 55.0° at a temperature of -170°C ± 1°C. A total of 192 oscillation images were collected. Data was swept using ω scan from 130.0° to 190° in 2.00° steps at χ = 45.0° and φ = 0.0°. The exposure rate was 80.0 [seconds/°]. The second sweep was performed using ω scan from 0.0° to 162.0° in 2.00° steps at χ = 45.0° and φ = 180.0°. The exposure rate was 80.0 [seconds/°]. Another sweep was performed using ω scan from 0.0° to 162.0° in 2.00° steps at χ = 45.0° and φ = 90.0°. The exposure rate was 80.0 [seconds/°]. The distance between the crystal and detector was 127.40 mm. Read out was performed at a 0.100 mm pixel mode.

### (Data reduction)

If 0 is unique among the collected 0 reflections (Rᵢₙₜ = 0.0115), equivalent reflections were consolidated.

The linear absorption coefficient µ for Mo-Kα was 0.875cm⁻¹. An empirical absorption correction that results in the transmission rate in the range of 0.958 to 0.992 was applied. The data was corrected for Lorentz and polarization effects.

### (Structural analysis and refinement)

The structure was analyzed using a direction method (SHELXT Version 2014/5: Sheldrick, G. M. (2014). Acta Cryst. A70, C1437) and expanded using Fourier technique. Non-hydrogen atoms were refined anistropically. Hydrogen atoms were refined by using a riding model. The final cycle of full-matrix least-squares refinement on F² converged by using the following unweighted and weighted match factors based on 8184 observed reflections and 501 variable parameters (greatest parameter shift was 0.00-fold of esd). $\begin{matrix}R 1 = \sum \left|\left|Fo\right|-\left|Fc\right|\right| / \sum \left|Fo\right| = 0.0285 \\ wR 2 = {\left[\sum \left(w{\left({Fo}^{2}-{Fc}^{2}\right)}^{2}\right) / \sum w {\left({Fo}^{2}\right)}^{2}\right]}^{1 / 2} = 0.0736\end{matrix}$

Least square function minimization: (SHELXL Version 2017/1)

Σw(Fₒ²-F_{c}²)² wherein w = least square weighting.

The goodness of fit was 1.06. The goodness of fit is defined as follows: ${\left[\sum w {\left({Fo}^{2}-{Fc}^{2}\right)}^{2} / \left(No-Nv\right)\right]}^{1 / 2}$ wherein No = number of observeations
Nv = number of variables.

Unit weighting was used. The maximum and minimum peaks of the final differential Fourier map corresponded to 0.31 and -0.20e⁻/Å³, respectively. The final Flack parameter (Parsons, S. and Flack, H. (2004), Acta Cryst. A60, s61) was 0.11 (11), showing that the absolute structure that was present was correct (Flack, H.D. and Bernardinelli (2000), J. Appl. Cryst. 33, 114-1148).

Neutral atomic scattering factors were taken from the International Table in Crystallography (IT), Vol. C, Table 6.1.1.4 (International Tables for Crystallography, Vol. C (1992). Ed. A.J.C. Wilson, Kluwer Academic Publishers, Dordrecht, Netherlands, Table 6.1.1.4, pp. 572). The anomalous dispersion effect was included in Fcalc (Ibers, J. A. & Hamilton, W. C.; Acta Crystallogr., 17, 781 (1964)); the values of Δf' and Δf" were from Creagh and McAuley (Creagh, D. C. & McAuley, W.J.; "International Tables for Crystallography", Vol C, (A.J.C. Wilson, ed.), Kluwer Academic Publishers, Boston, Table 4.2.6.8, pages 219-222 (1992)). The values of mass attenuation coefficient were from Creagh and Hubbell (Creagh, D. C. & Hubbell, J.H..; "International Tables for Crystallography", Vol C, (A.J.C. Wilson, ed.), Kluwer Academic Publishers, Boston, Table 4.2.4.3, pages 200-206 (1992).). All calculations used CrystalStructure (CrystalStructure 4.3: Crystal Structure Analysis Package, Rigaku Corporation (2000-2018). Tokyo 196-8666, Japan) crystallography software packages except for refinement using SHELXL Version 2017/1 (SHELXL Version 2017/1: Sheldrick, G. M. (2008). Acta Cryst. A64, 112-122).

Crystal data were as follows.

### [Numeral 3]

| | |
|---|---|
| **Empirical Formula** | **C₂₂H₂₉NO₄** |
| **Formula Weight** | **371.48** |
| **Crystal Color. Habit** | **colorless, prism** |
| **Crystal Dimensions** | **0.200 X 0.110 X 0.090 mm** |
| **Crystal System** | **triclinic** |
| **Lattice Type** | **Primitive** |
| **Lattice Parameters** | **a = 9.04561(19) A** |
| | **b = 10.3133(2) A** |
| | **c = 10.3424(2) A** |
| | **α = 91.615(7) °** |
| | **β = 93.663(7) °** |
| | **γ = 94.236(7) °** |
| | **V = 959.72(4) A³** |
| **Space Group** | **P1 (#1)** |
| **Z value** | **2** |
| **D_{calc}** | **1.285 g/cm³** |
| **F₀₀₀** | **400.00** |
| **µ(MoKα)** | **0.875 cm⁻¹** |

Intensity measurements were as follows.

### [Numeral 4]

| | |
|---|---|
| **Diffractometer** | **R-AXIS RAPID** |
| **Radiation** | **MoKα (λ = 0.71075 A) multi-layer mirror monochromated** |
| **Voltage. Current** | **50kV, 24mA** |
| **Temperature** | **-170.0°C** |
| **Detector Aperture** | **460.0 x 256.0 mm** |
| **Data Images** | **192 exposures** |
| **(·) oscillation Range (χ=45.0. ϕ=0.0)** | **130.0 - 190.0°** |
| **Exposure Rate** | **80.0 sec./°** |
| **(·) oscillation Range (χ=45.0, ϕ=180.0)** | **0.0 - 162.0°** |
| **Exposure Rate** | **80.0 sec./°** |
| **(·) oscillation Range (χ=45.0. ϕ=90.0)** | **0.0 - 162.0°** |
| **Exposure Rate** | **80.0 sec./°** |
| **Detector Position** | **127.40 mm** |
| **Pixel Size** | **0.100 mm** |
| **2θₘₐₓ** | **55.0°** |
| **No. of Reflections Measured** | **Total: 16466** |
| | **Unique: 8184 (Rᵢₙₜ = 0.0115)** |
| | **Parsons quotients (Flack x parameter):3584** |
| **Corrections** | **Lorentz-polarization** |
| | **Absorption** |
| | **(trans. factors: 0.958 - 0.992)** |

Structural analysis and refinement were the following. [Numeral 5]

| | |
|---|---|
| **Structure Solution** | **Direct Methods (SHELXT Version 2014/5)** |
| **Refinement** | **Full-matrix least-squares on F²** |
| **Function Minimized** | **Σ w(Fo² - Fc²)²** |
| **Least Squares Weights** | **w = 1/[σ²(Fo²) + (0.0446 · P)² + 0.1771 - P ]** |
| | **where P = (Max(Fo²,0) + 2Fc²)/3** |
| **2θₘₐₓ cutoff** | **55.0°** |
| **Anomalous Dispersion** | **All non-hydrogen atoms** |
| **No. Observations (All reflections)** | **8184** |
| **No. Variables** | **501** |
| **Reflection/Parameter Ratio** | **16.34** |
| **Residuals: R1 (I>2.00σ(I))** | **0.0285** |
| **Residuals: R (All reflections)** | **0.0293** |
| **Residuals: wR2 (All reflections)** | **0.0736** |
| **Goodness of Fit Indicator** | **1.060** |
| **Flack parameter (Parsons' quotients = 3584)** | **0.11(11)** |
| **Max Shift/Error in Final Cycle** | **0.000** |
| **Maximum peak in Final Diff. Map** | **0.31 e⁻/A³** |
| **Minimum peak in Final Diff. Map** | **-0.20 e⁻/A³** |

### (Example 11)

### X-ray powder diffraction (XRPD) on RS-Trolox•R-PEA (racemate), R-Trolox•R-PEA, and R-Trolox•R-PEA•NMP (solvate)

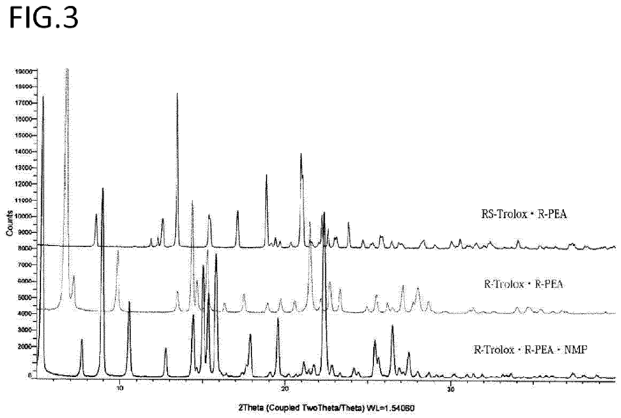

X-ray powder diffraction (XRPD) was performed on RS-Trolox•R-PEA (racemate), R-Trolox•R-PEA, and R-Trolox•R-PEA•NMP (solvate). Figure 3 and Table 6 show the results. X-ray powder diffraction (XRPD) was measured at room temperature under conditions of Cu Kα ray, X-ray tube current of 40 milliamperes, a voltage of 40 kilovolts, step of 0.015 degrees, and measurement time of 48 second/step in a diffraction angle 2θ range of 5 to 40 degrees by using Bruker AXS D8 ADVANCE.

**[Table 6-1]**

| **RS-Trolox ● R-PEA** | | | **R-Trolox ● R-PEA** | | | **R-Trolox ● R-PEA ● NMP** | | |
|---|---|---|---|---|---|---|---|---|
| **2θ** | **d Value** | **Gross Intensity** | **2θ** | **d Value** | **Gross Intensity** | **2θ** | **d Value** | **Gross Intensity** |
| **8.54** | **10.35** | **1964** | **6.75** | **13.08** | **17089** | **5.30** | **16.65** | **16540** |
| **10.89** | **8.12** | **153** | **7.19** | **12.29** | **2153** | **7.69** | **11.49** | **2385** |
| **11.87** | **7.45** | **647** | **9.86** | **8.97** | **3892** | **8.94** | **9.89** | **11334** |
| **12.31** | **7.18** | **694** | **13.49** | **6.56** | **1462** | **10.55** | **8.38** | **4723** |
| **12.55** | **7.05** | **1791** | **14.37** | **6.16** | **7065** | **11.51** | **7.68** | **215** |
| **13.45** | **6.58** | **9565** | **14.67** | **6.63** | **2080** | **12.78** | **6.92** | **1939** |
| **15.41** | **5.75** | **1980** | **15.29** | **5.79** | **3941** | **14.41** | **6.14** | **3846** |
| **17.11** | **5.18** | **2378** | **16.34** | **5.42** | **686** | **14.64** | **6.64** | **707** |
| **18.87** | **4.70** | **4647** | **17.51** | **5.06** | **1286** | **15.04** | **5.89** | **6968** |
| **19.67** | **4.51** | **504** | **18.94** | **4.68** | **701** | **15.35** | **5.77** | **5169** |
| **20.36** | **4.36** | **463** | **19.73** | **4.50** | **984** | **15.83** | **5.60** | **7505** |
| **21.00** | **4.23** | **4405** | **20.58** | **4.31** | **891** | **16.44** | **5.39** | **394** |
| **21.56** | **4.12** | **511** | **21.51** | **4.13** | **5716** | **17.44** | **5.08** | **398** |
| **22.19** | **4.00** | **2003** | **22.14** | **4.01** | **1027** | **17.75** | **4.99** | **944** |
| **22.57** | **3.94** | **1247** | **22.71** | **3.91** | **2054** | **17.86** | **4.96** | **2724** |
| **23.03** | **3.86** | **621** | **23.31** | **3.81** | **1610** | **19.07** | **4.65** | **471** |
| **23.83** | **3.73** | **1706** | **24.93** | **3.57** | **541** | **19.56** | **4.54** | **3794** |
| **24.69** | **3.60** | **615** | **25.49** | **3.49** | **1252** | **20.20** | **4.39** | **367** |
| **25.26** | **3.52** | **410** | **26.20** | **3.40** | **732** | **20.67** | **4.29** | **328** |
| **25.81** | **3.45** | **826** | **26.49** | **3.36** | **449** | **21.14** | **4.20** | **1131** |
| **26.43** | **3.37** | **532** | **27.10** | **3.29** | **1849** | **21.43** | **4.14** | **677** |
| **26.96** | **3.31** | **329** | **27.76** | **3.21** | **759** | **21.72** | **4.69** | **980** |
| **28.32** | **3.15** | **562** | **28.03** | **3.18** | **1726** | **22.34** | **3.98** | **10312** |
| **29.06** | **3.07** | **317** | **28.68** | **3.11** | **856** | **22.82** | **3.89** | **945** |
| **30.07** | **2.97** | **424** | **29.55** | **3.02** | **203** | **23.29** | **3.82** | **411** |
| **30.53** | **2.93** | **688** | **29.67** | **3.01** | **228** | **24.15** | **3.68** | **764** |

**[Table 6-2]**

| | | | | | | | | |
|---|---|---|---|---|---|---|---|---|
| 31.07 | 2.88 | 224 | 31.16 | 2.87 | 292 | 24.38 | 3.65 | 447 |
| 31.53 | 2.84 | 447 | 31.36 | 2.85 | 495 | 25.43 | 3.50 | 2315 |
| 32.02 | 2.79 | 261 | 31.97 | 2.80 | 232 | 25.61 | 3.48 | 1363 |
| 32.39 | 2.76 | 455 | 32.59 | 2.75 | 227 | 26.48 | 3.36 | 3365 |
| 33.32 | 2.69 | 151 | 34.01 | 2.63 | 564 | 26.92 | 3.31 | 760 |
| 33.67 | 2.66 | 155 | 34.69 | 2.58 | 541 | 27.10 | 3.29 | 490 |
| 34.08 | 2.63 | 560 | 34.76 | 2.58 | 496 | 27.47 | 3.24 | 1720 |
| 34.54 | 2.59 | 206 | 35.45 | 2.53 | 423 | 28.01 | 3.18 | 514 |
| 35.41 | 2.53 | 280 | 36.19 | 2.48 | 263 | 28.69 | 3.11 | 517 |
| 35.85 | 2.50 | 175 | 36.73 | 2.45 | 339 | 29.16 | 3.06 | 359 |
| 36.28 | 2.47 | 220 | 36.97 | 2.43 | 241 | 29.47 | 3.03 | 336 |
| 37.33 | 2.41 | 446 | 38.85 | 2.32 | 172 | 30.20 | 2.96 | 361 |
| 38.23 | 2.35 | 236 | 39.37 | 2.29 | 238 | 30.99 | 2.88 | 364 |
| 39.26 | 2.29 | 265 | | | | 31.38 | 2.85 | 421 |
| | | | | | | 31.90 | 2.80 | 382 |
| | | | | | | 32.46 | 2.76 | 263 |
| | | | | | | 32.78 | 2.73 | 264 |
| | | | | | | 33.22 | 2.69 | 309 |
| | | | | | | 33.63 | 2.66 | 470 |
| | | | | | | 34.11 | 2.63 | 236 |
| | | | | | | 34.43 | 2.60 | 252 |
| | | | | | | 35.06 | 2.56 | 306 |
| | | | | | | 35.41 | 2.53 | 318 |
| | | | | | | 35.79 | 2.51 | 349 |
| | | | | | | 36.14 | 2.48 | 310 |
| | | | | | | 37.40 | 2.40 | 494 |
| | | | | | | 38.07 | 2.36 | 361 |
| | | | | | | 38.86 | 2.32 | 389 |
| | | | | | | 39.26 | 2.29 | 239 |

### (Example 12)

### Double crystallization using DMP/toluene mixture solvent (first step: optical resolution, second step: recrystallization)

At room temperature, DMP (50.0 g) and toluene (200.0 g) were added to Trolox (25.00 g), and the temperature was raised to 50°C. R-PEA (7.26 g) was added, and the solution was cooled to 45°C, and then an R-Trolox•R-PEA (0.03 g) seed crystal was seeded and incubated for 2 hour. After adding R-PEA (3.03 g) dropwise over 20 minutes, the mixture was incubated for 40 minutes. After cooling the mixture to 5°C, the precipitate was filtered out. The precipitate was washed twice with a DMP/toluene mixture solvent (10.0 g/40.0 g), washed twice with toluene (50 g), and then dried to obtain a DMP solvate of R-Trolox•R-PEA (yield: 19.32 g, percentage yield 40.9%, optical purity: 94.7%ee).

To the crystal obtained above (18.00 g), DMP (54.0 g) and toluene (162.0 g) were added, and the temperature was raised to 80°C. The solution was then cooled to 70°C, and an R-Trolox•R-PEA (0.02 g) was seeded and incubated for 1 hour, and cooled to 5°C, and then the precipitate was filtered out. The precipitate was washed with a DMP/toluene mixture solvent (6.8 g/20.3 g), washed twice with toluene (27.0 g), and then dried to obtain a DMP solvate of R-Trolox•R-PEA (yield: 16.82 g, percentage yield 93.4%, optical purity: 99.9%ee).

### (Example 13)

### Optical resolution for obtaining S-Trolox•S-PEA•NMP

At room temperature, NMP (50.00 g) and toluene (200.0 g) were added to Trolox (25.00 g), and the temperature was raised to 50°C. S-PEA (7.26 g) was added. The mixture was cooled to 45°C, then an S-Trolox•S-PEA (0.03 g) seed crystal was seeded and incubated for 2 hours. After adding S-PEA (3.03 g) dropwise over 30 minutes, the mixture was incubated for 2 hours. The mixture was cooled to 0°C and incubated overnight, and then the precipitate was filtered out. The precipitate was washed twice with an NMP/toluene mixture solvent (10.0 g/40.0 g), washed twice with toluene (50 g), and then dried to obtain an NMP solvate of S-Trolox•S-PEA (yield: 19.32 g, percentage yield 41.1%, optical purity: 97.0%ee).

### (Example 14A)

### Preparation of (R)-6-hydroxy-2,5,7,8-tetramethylchroman-2-carboxylic acid

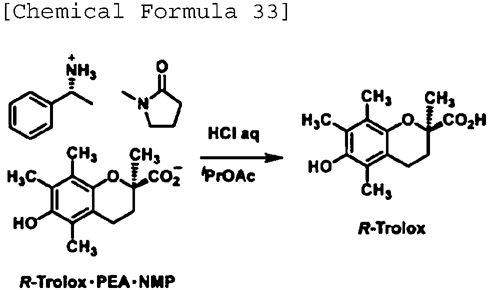

After adding isopropyl acetate (110 kg) and water (10.99 kg) to R-Trolox•PEA•NMP (10.00 kg, 21.25 mol), 35% hydrochloric acid (3.80 kg, 36.55 mol) was added dropwise and stirred for 20 minutes at 25 ± 5°C. After the mixture was left standing and separated, the organic layer was washed twice with water (15.80 kg, 15.83 kg) and concentrated under reduced pressure at 45°C or lower (amount recovered: 92.0 kg). Isopropyl acetate (8.38 kg) was added to prepare an isopropyl acetate solution of R-Trolox (16.7%, 31.9 kg). This was used in the following step (assuming yield of 100%) .

### (Example 14B)

### Preparation of (R)-6-hydroxy-2,5,7,8-tetramethylchroman-2-carboxylic acid

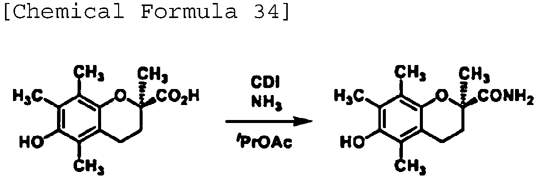

An isopropyl acetate solution of R-Trolox (16.7%, 31.8 kg, 21.25 mol (assumed)) was added dropwise into a slurry of isopropyl acetate (26.6 kg) added with CDI (Hodogaya Chemical) (8.61 kg, 52.12 mol) over 1 hour at 20 ± 5°C. The mixture was washed with isopropyl acetate (2.94 kg) and incubated for 2 hours. While the temperature was maintained at less than 15 ± 5°C, the isopropyl acetate solution was added dropwise into 28% ammonium water (Nacalai Tesque, 16.15 kg, 265.61 mol) that had been precooled to 15 ± 5°C over an hour. The mixture was washed with isopropyl acetate (1.33 kg). After stirring for 1.5 hours, isopropyl acetate (43.5 kg) and water (21.27 kg) were added. An aqueous phosphoric acid solution prepared by mixing water (10.64 kg) and 85% phosphoric acid (19.60 kg) was added dropwise at 10 to 60 °C. The mixture was washed with water (1.06 kg) and separated to obtain an isopropyl acetate solution (109.09 kg) of the title compound, which was used in the following step (assuming yield of 100%).

### (Example 14C)

### Preparation of (R)-2-hydroxy-2-methyl-4-(2,4,5-trimethyl-3,6-dioxocyclohexa-1,4-dienyl)butanamide

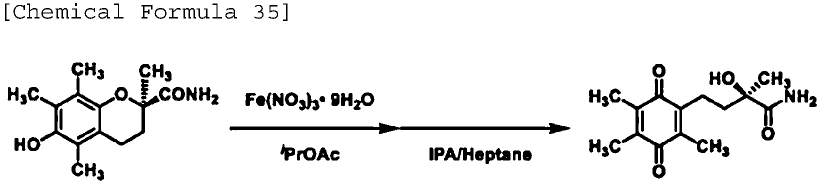

An aqueous 33.9% ferric nitrate solution (Kokusan Chemical) (33.64 kg, 48.66 mol) was added dropwise into an isopropyl acetate solution of (R)-6-hydroxy-2,5,7,8-tetramethylchroman-2-carboxylic acid (109.58 kg, 21.25 mol (assumed)) over one hour at 30°C or less. The mixture was incubated for 2.5 hours at 25 ± 5°C, then added with salt (3.71 kg), stirred for 15 minutes, and left standing. After separation, the mixture was heated to 40 ± 5°C, added with aqueous 12.1% citric acid solution (13.52 kg), and washed. After further adding an aqueous 10% sodium carbonate solution (13.24 kg) and washing, the mixture was washed with water (10.60 kg). The organic layer obtained by separation was concentrated under reduced pressure at 45°C or less (amount recovered: 52.7 kg). After raising the temperature to 80 ± 5°C and incubating for 1 hour, a seed crystal (0.003 kg) was added and incubated for 1 hour. After cooling to 0°C at a cooling rate of 10°C/hour and incubating for 11 hours, the precipitate was filtered out and washed twice with pre-cooled isopropyl acetate (13.24 kg × 2). The precipitate was aerated and dried with nitrogen at 45°C or less to obtain a crude crystal (yield: 4.47 kg, percentage yield of 3 steps: 79.3%, quality: 100.00%, optical purity: > 99.9%ee).

The resulting crude crystal (4.01 kg) was recrystallized with an isopropanol/heptane mixture solvent to obtain the title compound (3.77 kg, recrystallization yield: 94.0%, quality: 100.00%, optical purity: > 99.9%ee).

As described above, the present disclosure is exemplified by the use of its preferred embodiments. It is understood that the scope of the present disclosure should be interpreted solely based on the Claims.

### [Industrial Applicability]

The present disclosure is useful for the manufacture of an optically active medicament.

## Claims

1. A method of manufacturing a solid salt of a compound of formula I, comprising adding an amide-based solvent in the presence of an optical resolving agent to a sample comprising the compound of formula I and suspected of comprising a compound of formula II: 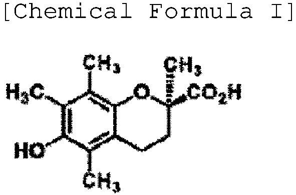
Formula I: (R)-6-hydroxy-2,5,7,8-tetramethylchroman-2-carboxylic acid (hereinafter R Trolox) 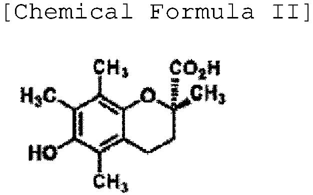
Formula II: (S)-6-hydroxy-2,5,7,8-tetramethylchroman-2-carboxylic acid (hereinafter S Trolox),
wherein the solid salt is an amide-based solvate,
wherein the optical resolving agent is selected from phenylethylamine, N-methylglucamine, arginine, lysine, pseudoephedrine, leucinol, and aminophenyl propanol,wherein the amide-based solvent is one or more solvents selected from N-methylpyrrolidone (NMP), dimethylacetamide (DMA), and N,N-dimethylformamide (DMF).

2. The method of claim 1, wherein the optical resolving agent comprises at least an asymmetric carbon, and a moiety that can form a salt with the R Trolox or S Trolox.

3. The method of claim 1 or 2, comprising adding the optical resolving agent to the sample.

4. The method of any one of the preceding claims, wherein addition of the amide-based solvent comprises adding a mixture of the optical resolving agent and the amide-based solvent to the sample.

5. The method of any one of the preceding claims, wherein the sample comprises a complex with the optical resolving agent.

6. The method of any one of the preceding claims, wherein the sample comprises a mixture of a compound of formula I and a compound of formula II.

7. The method of any one of the preceding claims, wherein an enantiomeric excess of a manufactured solid salt of a compound of formula I is at least 98% or greater, preferably at least 99% or greater.

8. The method of any one of the preceding claims, further comprising dissolving the phenylethylamine into toluene.

9. The method of any one of the preceding claims, comprising adding a poor solvent, preferably toluene or ethyl acetate, to the sample.

10. The method of any one of the preceding claims, further comprising removing the amide-based solvent.

## Patentansprüche

1. Verfahren zum Herstellen eines festen Salzes einer Verbindung der Formel I, umfassend Hinzufügen eines Lösungsmittels auf Amidbasis in der Gegenwart eines optischen Auflösungsmittels zu einer Probe, die die Verbindung der Formel I umfasst und von der vermutet wird, dass sie eine Verbindung der Formel II umfasst: 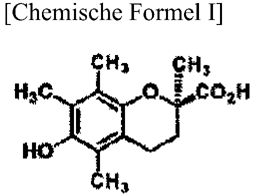
Formel I: (R)-6-Hydroxy-2,5,7,8-tetramethylchroman-2-carbonsäure (im Folgenden R-Trolox), 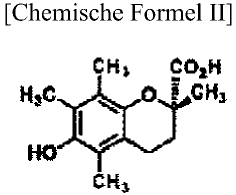
Formel II: (S)-6-Hydroxy-2,5,7,8-tetramethylchroman-2-carbonsäure (im Folgenden S-Trolox), wobei das feste Salz ein Solvat auf Amidbasis ist,
wobei das optische Auflösungsmittel ausgewählt ist aus Phenylethylamin, N-Methylglucamin, Arginin, Lysin, Pseudoephedrin, Leucinol und Aminophenylpropanol, wobei das Lösungsmittel auf Amidbasis ein oder mehrere Lösungsmittel ist, die ausgewählt sind aus N-Methylpyrrolidon (NMP), Dimethylacetamid (DMA) und N,N-Dimethylformamid (DMF).

2. Verfahren nach Anspruch 1, wobei das optische Auflösungsmittel mindestens einen asymmetrischen Kohlenstoff und eine Einheit, die mit dem R-Trolox oder S-Trolox ein Salz bilden kann, umfasst.

3. Verfahren nach Anspruch 1 oder 2, umfassend Hinzufügen des optischen Auflösungsmittels zu der Probe.

4. Verfahren nach einem der vorhergehenden Ansprüche, wobei die Hinzufügung des Lösungsmittels auf Amidbasis Hinzufügen einer Mischung aus dem optischen Auflösungsmittel und dem Lösungsmittel auf Amidbasis zu der Probe umfasst.

5. Verfahren nach einem der vorhergehenden Ansprüche, wobei die Probe einen Komplex mit dem optischen Auflösungsmittel umfasst.

6. Verfahren nach einem der vorhergehenden Ansprüche, wobei die Probe eine Mischung aus einer Verbindung der Formel I und einer Verbindung der Formel II umfasst.

7. Verfahren nach einem der vorhergehenden Ansprüche, wobei ein Enantiomerenüberschuss eines hergestellten festen Salzes einer Verbindung der Formel I mindestens 98 % oder mehr, vorzugsweise mindestens 99 % oder mehr beträgt.

8. Verfahren nach einem der vorhergehenden Ansprüche, ferner umfassend Auflösen des Phenylethylamins in Toluol.

9. Verfahren nach einem der vorhergehenden Ansprüche, umfassend Hinzufügen eines schlechten Lösungsmittels, vorzugsweise Toluol oder Ethylacetat, zu der Probe.

10. Verfahren nach einem der vorhergehenden Ansprüche, ferner umfassend Entfernen des Lösungsmittels auf Amidbasis.

## Revendications

1. Procédé de fabrication d'un sel solide d'un composé de formule I, comprenant l'ajout d'un solvant à base d'amide en présence d'un agent de résolution optique à un échantillon comprenant le composé de formule I et suspecté de comprendre un composé de formule II : 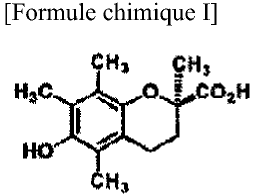
Formule I : acide (R)-6-hydroxy-2,5,7,8-tétraméthylchroman-2-carboxylique (ci-après R-Trolox) 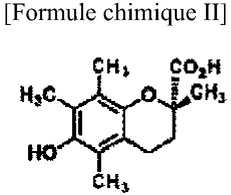
Formule II : acide (S)-6-hydroxy-2,5,7,8-tétraméthylchroman-2-carboxylique (ci-après S-Trolox) dans lequel le sel solide est un solvate à base d'amide,
dans lequel l'agent de résolution optique est choisi parmi la phényléthylamine, la N-méthylglucamine, l'arginine, la lysine, la pseudoéphédrine, le leucinol et l'aminophénylpropanol, dans lequel le solvant à base d'amide est un ou plusieurs solvants choisis parmi la N-méthylpyrrolidone (NMP), le diméthylacétamide (DMA) et le N,N-diméthylformamide (DMF).

2. Procédé de la revendication 1, dans lequel l'agent de résolution optique comprend au moins un carbone asymétrique et un groupement pouvant former un sel avec le R-Trolox ou le S-Trolox.

3. Procédé de l'une des revendications 1 ou 2, comprenant l'ajout de l'agent de résolution optique à l'échantillon.

4. Procédé de l'une quelconque des revendications précédentes, dans lequel l'ajout du solvant à base d'amide comprend l'ajout d'un mélange de l'agent de résolution optique et du solvant à base d'amide à l'échantillon.

5. Procédé de l'une quelconque des revendications précédentes, dans lequel l'échantillon comprend un complexe avec l'agent de résolution optique.

6. Procédé de l'une quelconque des revendications précédentes, dans lequel l'échantillon comprend un mélange d'un composé de formule I et d'un composé de formule II.

7. Procédé de l'une quelconque des revendications précédentes, dans lequel un excès énantiomérique d'un sel solide fabriqué d'un composé de formule I est d'au moins 98 % ou plus, de préférence d'au moins 99 % ou plus.

8. Procédé de l'une quelconque des revendications précédentes, comprenant en outre la dissolution de la phényléthylamine dans le toluène.

9. Procédé de l'une quelconque des revendications précédentes, comprenant l'ajout d'un mauvais solvant, de préférence du toluène ou de l'acétate d'éthyle, à l'échantillon.

10. Procédé de l'une quelconque des revendications précédentes, comprenant en outre l'élimination du solvant à base d'amide.
